# EUROPEAN PATENT APPLICATION

(11) **EP 1 671 639 A1**
(43) Date of publication of application: **21.06.2006**
(21) Application number: 05111924.6
(22) Date of filing: 09.12.2005
(51) Int. Cl.: A61K 31/737, A61K 45/06, A61P 31/18

(54) **Treatment of acquired immunedeficiency syndrome AIDS with a combination therapy including curdlan sulphate**

(30) Priority: 09.12.2004 US 634571
(71) Applicant: MAXWELL, Gordon, New York, NY 10028 (US)
(72) Inventor: Yutaro, Kaneko, N.Y. 10028, NEW YORK (US); Maxwell, Gordon, N.Y. 10028, NEW YORK (US)
(74) Representative: De Gregori, Antonella

(57) **Abstract**

HIV infections are treated with sequential treatment regimens comprising a first regimen which is a combination of highly active antiretroviral therapy drugs selected from the group consisting of nucleoside reverse transcriptase inhibitors, non-nucleoside reverse transcriptase inhibitors and protease inhibitors to reduce the HIV levels; and a second treatment regimen which is administered after said HIV levels are reduced or resistance is observed, said second treatment regimen an amount of an HIV entry/fusion inhibitor to form a modified treatment regimen and continuing to administer said modified treatment regimen for a period of from 10 to 30 days.

## Description

### FIELD OF INVENTION

The invention provides a means for treating AIDS. In the last twenty years, the human immunodeficiency virus (HIV) has been discovered to be the cause of AIDS. Many drugs have been approved for the treatment of AIDS which exhibit activity due to their ability to inhibit replication of HIV. The administration of combinations of these drugs which are the basis of highly active antiretinoviral therapy (HAART) has been found to reverse the ravages of AIDS and to permit patients to is directed enjoy a nearly normal lifestyle. When the HAART drugs are discontinued, the levels of HJIV begin to rise from undetectable blood levels to pre-treatment levels. The consensus of the art is that the HAART drugs do not reach a biological reservoir which means that drug therapy is a life-long necessity. The selection of the most effective combination of drugs for a particular patient is somewhat difficult as the 20 approved drugs when given in two, three or four different combinations gives rise to thousands of possible combinations. Commonly used combinations include:

Nucleoside reverse transcriptase inhibitors (NRTI) and non-nucleoside reverse transcriptase inhibitors:
1. efavirenz (400mg/d) + lamivudine (300mg/d) + zidovudine (200mg/tid) or tenifovir (300mg/d) or stavudine(40mg/tid)
2. efavirenz(400mg/d) + lamivudine(300mg/d) + didanosine (400mg/d)
3. nevirapine (200-400mg/d) + lamivudine(300mg/d) + zidovudine(200mg/tid) or stavuduine(40mg tid) or didanosine (400mg tid)
4. abacavir(300mg/d) + lamivudine (300mg/d) + zidovudine(200mg tid Protease inhibitor based regimens:
   1. lopinavir (400mg/bid) + ritonavir(100mgbid) + lamivuidine (300mg/d) + zidovudine(200mg tid) or stavudine (40mgbid)
   2. amprenavir(1200mg) + ritonavir (100-200mg/d) + lamivudine (300mg/d) + zidovudine (200mgbid) or stavudine (40mg/tid)
   3. indinavir(8900mg tid) + ritinovir (100-200mg/d) + lamivudine (300mg/d + ziodovudine (200mg/d) or stavudine (40mg/tid)
   4. saquinavir(1200mg tid + ritinavir(100-200mg/d) + lamivudine (300mg/d) + zidovudine(200mg/tid) or stavudine (40mg tid)

A different class of HIV drugs comprises the entry/fusion inhibitors which appear to block the entry or fusion of HIV with T-cells of the human immune system. These drugs include enfuvirtide and curdlan sulfate.

### SUMMARY OF THE INVENTION

The present invention comprises treating first treating a patient with an HIV infection with an effective amount of an effective combination of a treatment regimen comprising one or more HAART drugs selected from the group consisting of nucleoside reverse transcriptase inhibitors, non-nucleoside reverse transcriptase inhibitors and protease inhibitors to reduce the HIV levels and thereafter adding to said treatment regimen an effective amount amount of an HIV entry/fusion inhibitor to form a modified treatment regimen and continuing to administer said modified treatment regimen for a period of from 10 to 30 days. The effective amount of a treatment regimen of one or more HAART drugs will comprise the conventional dose.

After an initial treatment period of e.g. 21 days, the viral load is measured daily and if the viral load rises to measurable levels for three days, the HAART therapy may be resumed.

A preferred embodiment of the invention comprises the initial administration of a combination of HAART drugs until HIV is undetectable and thereafter adding an entry/fusion inhibitor such as curdlan sulfate to the treatment regimen.

The invention also comprises a method of treating human immunodeficiency virus (HIV) infections which comprises treating a patient with an HIV infection which is resistant to a treatment regimen comprising the administration of a combination of highly active antiretroviral therapy drugs selected from the group consisting of nucleoside reverse transcriptase inhibitors, non-nucleoside reverse transcriptase inhibitors and protease inhibitors said method comprising administering an amount of an HIV entry/fusion inhibitor for a period of from 10 to 30 days. The preferred entry/fusion inhibitor is curdlan sulfate.

### DETAILED DESCRIPTION OF THE INVENTION

The present invention provides a treatment method for HIV infections that is designed to completely eradicate the HIV virus. The prior art therapy regimens have been found to be effective in the elimination of detectable levels of the HIV virus for as long as drug therapy is maintained. The reappearance of HIV virus in the blood after the interruption of current therapies makes it likely that the HIV virus is capable of being retained in some biological reservoir where current therapies are unable to create an environment that inhibits or destroys the HIV virus. The administration of the entry/fusion inhibitor curdlan sulfate by itself has been shown to induce large increases in detectable bloods levels of T-cells when it is administered to HIV infected patients. The large increase in T-cells is believed to be due to a release or flushing of T-cells from a biological repository or storage site rather than a biological production of new T-cells. This phenomenon has caused the applicants to theorize that the repository or storage site for the T-cells is also a sanctuary for the HIV virus which appears to have an ability to shield itself from the effects of anti-HIV drugs which are circulated in the blood to all of the bodies organs. The invention is directed to the administration of an entry/fusion inhibitor after a HAART drug combination has been administered in an effective amount for a sufficient time to cause the HIV cells to been eradicated from the blood stream. Thereafter, the entry/fusion inhibitor is administered in an effective amount for a sufficient period of time to eradicate the HIV cells.

Any of the conventionally employed doses of anti-AIDS drugs may used to eliminate detectable levels of HIV cells from a patients blood. At that point an entry/fusion inhibitor such as curdlan sulfate is administered according to one or more of the following dosage schedules:
1. 50-200mg curdlan sulfate administered intravenously over 30 minutes once a day for 21 days.
2. 50-200mg curdlan sulfate administered intravenously over 30 minutes every 12 hours for 21 days
3. 10-20mg/hour of curdlan sulfate administered IV continuously for 21 days.

Curdlan sulfate is described in U.S. 5,512,672, which is incorporated by reference. This product is chemically identified as a sulfated curdlan (a beta-1,3-D-glucan) having a sulfur content of about 12.4 to 17% and an average molecular weight by gel filtration of from about 27,000-330,000. Curdlan sulfate may be formulated for intravenous use by dissolving the product vial in 5 ml of 5% glucose solution and then diluting with 100 ml of 5% glucose to form an intravenous formulation.

### EXAMPLE 1

Patients are selected for treatment with the method of the invention based on the fact that they have previously tested positive for HIV and presently have some detectable level of HIV. Initially, the patients are screened for the effect of curdlan sulfate on coagulation times by measuring the time to coagulation using the activated partial thromboplastin time test (APTT) after IV infusion of 50mg of curdlan sulfate in over a period of 30 minutes. A test dose of 50m of curdlan sulfate is given on the first day and each day thereafter, the dose of curdlan sulfate is increased in 50mg increments until the APTT is doubled. When the APTT is doubled, the maximum tolerated dose (MTD)of curdlan sulfate has been reached which is usually 200mg/d. although no bleeding at the site of injection or elsewhere is detected. When the MTD is determined for each patient, the MTD is repeated daily for 21 days while the particular dosage regimen of the HAART drugs is continued. After the 21 days of curdlan sulfate therapy is completed, the HAART treatment regimen is also suspended and the viral load is determined daily. If there is no measurable viral load after this treatment, the treatment is deemed to be successful. If the viral load returns to measurable levels for three days, the HAART therapy may be reinstituted followed by another sequence of curdlan sulfate therapy.

### EXAMPLE 2

Patients are selected for treatment using the same protocol that is applied in Example 1. The curdlan sulfate therapy is employed as in Example 1 except that a dose of 50mg IV is given every 12 hours for 21 days after the MTD is established as in Example 1. After the 21 days of curdlan sulfate therapy is completed, the HAART treatment regimen is also suspended and the viral load is determined daily. If there is no measurable viral load after this treatment, the treatment is deemed to be successful.

### EXAMPLE 3

Patients are selected for treatment using the same protocol that is applied in Example 1. The curdlan sulfate therapy is employed IV by continuous infusion at a rate of 10 mg per hour, using an infusion pump while the APTT is determined every 8-10 hours and if the APTT does not double the dose is increased to 15mg/hour and after 8-10 hours at this dose, the APTT is again determined and if the APTT has not doubled, the dose is increased to 20mg/hour and continued for 21 days while continuing the HAART therapy. At the end of 21 days, the HAART therapy is discontinued and the viral load is determined daily. If there is no measurable viral load after this treatment, the treatment is deemed to be successful.

### EXAMPLE 4

Patients are selected for treatment who no longer respond to HAART therapy and thus have elevated viral levels. These patients are tested for tolerance to once daily curdlan sulfate therapy as described in Example 1 and are then given the MTD of curdlan sulfate using the same protocol that is applied in Example 1. If there is no measurable viral load after this treatment, the treatment is deemed to be successful.

### EXAMPLE 5

Patients who fail to respond to once daily therapy as described in Example 4 are treated with curdlan sulfate at the MTD dose intravenously every 12 hours for 21 days. If there is no measurable viral load after this treatment, the treatment is deemed to be successful.

### EXAMPLE 6

Patients who fail to respond to once daily therapy as described in Example 5 are treated with curdlan sulfate continuously at the MTD dose as in Example 3. If there is no measurable viral load after this treatment, the treatment is deemed to be successful.

## Claims

1. A method of treating human immunodeficiency virus (HIV) infections which comprises:
(a) treating a patient with an HIV infection with a treatment regimen comprising an effective combination of highly active antiretroviral therapy drugs selected from the group consisting of nucleoside reverse transcriptase inhibitors, non-nucleoside reverse transcriptase inhibitors and protease inhibitors to reduce the HIV levels; and
(b) after said HIV levels are reduced, adding to said treatment regimen an amount of an HIV entry/fusion inhibitor to form a modified treatment regimen and continuing to administer said modified treatment regimen for a period of from 10 to 30 days.

2. A method of treating human immunodeficiency virus (HIV) infections as defined in claim 1 where the entry/fusion inhibitor is curdlan sulfate.

3. A method of treating human immunodeficiency virus (HIV) infections as defined in claim 1 wherein curdlan sulfate is administered at a level of 50-200mg intravenously over 30 minutes once a day for 21 days.

4. A method of treating human immunodeficiency virus (HIV) infections as defined in claim 1 wherein curdlan sulfate is administered at a level of 50-200mg intravenously over 30 minutes every 12 hours for 21 days.

5. A method of treating human immunodeficiency virus (HIV) infections as defined in claim 1 wherein curdlan sulfate is continuously administered at a level of 10-20mg/hour intravenously for 21 days.

6. A method of treating human immunodeficiency virus (HIV) infections as defined in claim 1 where step (a) has reduced the HIV level to an undetectable level.

7. A method of treating human immunodeficiency virus (HIV) infections as defined in claim 1 where step (a) has reduced the HIV level to detectable levels.

8. A method of treating human immunodeficiency virus (HIV) infections which comprises treating a patient with an HIV infection which is resistant to a treatment regimen comprising a combination of highly active antiretroviral therapy drugs selected from the group consisting of nucleoside reverse transcriptase inhibitors, non-nucleoside reverse transcriptase inhibitors and protease inhibitors said method comprising administering an amount of an HIV entry/fusion inhibitor for a period of from 10 to 30 days.

9. A method of treating human immunodeficiency virus (HIV) infections as defined in claim 9 where the entry/fusion inhibitor is curdlan sulfate.

10. A method of treating human immunodeficiency virus (HIV) infections as defined in claim 9 wherein curdlan sulfate is administered at a level of 50-200mg intravenously over 30 minutes once a day for 21 days.

11. A method of treating human immunodeficiency virus (HIV) infections as defined in claim 9 wherein curdlan sulfate is administered at a level of 50-200mg intravenously over 30 minutes every 12 hours for 21 days.

12. A method of treating human immunodeficiency virus (HIV) infections as defined in claim 9 wherein curdlan sulfate is continuously administered at a level of 10-20mg/hour intravenously for 21 days.
